# EUROPEAN PATENT SPECIFICATION

(11) **EP 4 539 911 B1**
(45) Date of publication and mention of the grant of the patent: **20.08.2025**
(21) Application number: 24727440.0
(22) Date of filing: 24.05.2024
(51) Int. Cl.: A61M 16/00, A61B 5/087

(54) **SYSTEM FOR CONTROLLING A SLEEP DISORDERED BREATHING THERAPY DEVICE**
SYSTEM ZUR STEUERUNG EINER ATEMTHERAPIEVORRICHTUNG MIT SCHLAFSTÖRUNGEN
SYSTÈME DE COMMANDE D'UN DISPOSITIF DE THÉRAPIE DE TROUBLE RESPIRATOIRE DU SOMMEIL

(30) Priority: 01.06.2023 EP 23176712
(43) Date of publication of application: 23.04.2025
(73) Proprietor: Koninklijke Philips N.V., 5656 AG Eindhoven (NL)
(72) Inventor: KAHLERT, Joachim, Johannes, 5656 AG Eindhoven (NL); HENDRIKS, Cornelis, Petrus, 5656 AG Eindhoven (NL); VAN ZANTEN, Joyce, 5656 AG Eindhoven (NL); FERREIRA DOS SANTOS DA FONSECA, Pedro, Miguel, 5656 AG Eindhoven (NL); VAN DEN ENDE, Daan, Anton, 5656 AG Eindhoven (NL)
(74) Representative: Philips Intellectual Property & Standards
(86) International application number: PCT/EP2024/064376
(87) International publication number: WO 2024/245935

(56) References cited:
- US-A1- 2010 174 161
- US-A1- 2013 072 999
- US-A1- 2022 203 063
- US-B1- 7 942 824

## Description

### FIELD OF THE INVENTION

The invention relates to the field of sleep disordered breathing therapy devices.

### BACKGROUND OF THE INVENTION

Sleep-disordered breathing (SDB) conditions, such as obstructive sleep apnea (OSA) and central sleep apnea (CSA), are becoming increasingly common. SDB conditions are often treated using SDB therapy devices, such as positive airway pressure (PAP) devices, mandibular advancement devices (MAD) and positional therapy devices.

PAP devices provide pressurized air to a subject to keep the subject's airways open, thus reducing SBD events. However, in subjects with cardiac comorbidities, the positive pressure of the PAP device can cause changes in the intrathoracic pressure, which in turn may alter various cardiac parameters (e.g. heart rate, blood pressure, cardiac filling, ejection rate, stroke volume and cardiac output). For example in chronic heart failure patients, the changed intrathoracic pressure leads to a shift in the venous return flow, the atrial filling and the stroke volume.

The change in cardiac parameters activates baroreceptors, leading to arousals. As a result, subjects with SDB and a cardiac comorbidity often experience an increased latency to fall asleep and insomnia. The decrease in sleep quality that such subjects experience often results in a decreased adherence to the prescribed PAP therapy, as subjects will often choose to reduce their use of the PAP device (e.g. using the PAP device for fewer hours in a night than is recommended), or even stop using the PAP device entirely, if they perceive that its use results in worse sleep.

There is therefore a need for improved SDB therapy, in particular, for subjects with SDB and a cardiac comorbidity.

US patent application US 7942824 A1 discloses an integrated sleep diagnosis and treatment device, and more particularly an integrated apnea diagnosis and treatment device. The sleep disorder treatment system can use a diagnosis device to perform various forms of analysis to determine or diagnose a subject's sleeping disorder or symptoms of a subject's sleep disorder.

### SUMMARY OF THE INVENTION

The invention is defined by the claims.

According to examples in accordance with an aspect of the invention, there is provided a processing system for controlling a sleep disordered breathing, SDB, therapy device providing therapy to a subject during a sleep session of the subject, the processing system being configured to: receive a first signal responsive to the subject's breathing during the sleep session; receive a second signal responsive to baroreceptor-initiated changes in sympathetic activity of the subject; process the first and second signals to detect a change in sympathetic activity; in response to the detected change in sympathetic activity exceeding a predetermined activity threshold: process the first and second signals to determine whether the detected change in sympathetic activity is chemoreceptor-initiated or baroreceptor-initiated; and generate a control signal for controlling the SDB therapy device, based on the determination as to whether the detected change in sympathetic activity is chemoreceptor-initiated or baroreceptor-initiated.

By generating the control signal based on whether a change in sympathetic activity of the subject is chemoreceptor-initiated or baroreceptor-initiated, an adjustment may be made to the SDB therapy device that results in fewer arousals, thus improving a quality of the subject's sleep.

The inventors have recognized that a subject who experiences cardiac stress during typical use of an SDB therapy device will experience improved sleep quality if the therapy provided by the device is adjusted to result in a slight increase in SBD events but a decrease in cardiac stress. **In** other words, the invention aims to provide a balance between reducing SDB events and limiting baroreceptor-initiated arousals.

**In** some examples, the SDB therapy device is a positive airway pressure, PAP, device; wherein the control signal comprises a first control signal, wherein the processing system is configured to: determine an adjusted pressure to be provided by the PAP device, based on the determination as to whether the detected change in sympathetic activity is chemoreceptor-initiated or baroreceptor-initiated; and generate the first control signal to control the PAP device to provide air to the subject at the adjusted pressure.

The invention is particularly beneficial for use with PAP devices, because the pressure delivered by a PAP device can cause cardiac stress (and therefore baroreceptor-initiated sympathetic activity and arousals). Baroreceptors are located in the cardiac chambers and in the vascular structures. A receptor activation initiates a sympathetic response. Strong sympathetic responses may initiate arousals.

By recognizing that the subject is experiencing baroreceptor-initiated sympathetic activity, an adjusted pressure that will reduce baroreceptor-initiated sympathetic activity may be determined, in order to reduce a likelihood of baroreceptor-initiated arousals. Similarly, if the subject is experiencing chemoreceptor-initiated sympathetic activity, an adjusted pressure that will reduce SDB events may be determined.

By automatically controlling the PAP device, adjustments to the pressure of the PAP device may be made in real-time, in an attempt to keep sympathetic activity below levels that lead to arousal. **In** this way, the pressure provided by the PAP device may be adjusted throughout a sleep session in order to reduce a likelihood of arousals during the sleep session and thus improve sleep quality.

**In** some examples, in response to a determination that the detected change in sympathetic activity is chemoreceptor-initiated, the adjusted pressure is an increased pressure; and in response to a determination that the detected change in sympathetic activity is baroreceptor-initiated, the adjusted pressure is a decreased pressure.

The inventors have recognized that chemoreceptor-initiated sympathetic activity is a result of a respiratory deficiency, indicating inadequate air pressure from the PAP device, while baroreceptor-initiated activity is due to an excessively high pressure causing cardiac stress. Chemoreceptors are located in the vascular structures of the lung. Chemoreceptors are activated responsive to an insufficient uptake of oxygen or an insufficient release of carbon dioxide.

Therefore, arousals may be minimized by increasing the pressure of the PAP device in response to chemoreceptor-initiated sympathetic activity and decreasing the pressure in response to baroreceptor-initiated sympathetic activity.

**In** some examples, the processing system is configured to: generate the first control signal configured to control the PAP device to provide air to the subject at the adjusted pressure for a predetermined time period; and in response to the predetermined time period elapsing, generate a second control signal configured to control the PAP device to provide air to the subject at a previous pressure.

The predetermined time period may, for example, be measured in terms of absolute time or may be a predetermined number of breathing cycles. The previous pressure may be the pressure at which the PAP device provided air to the subject immediately prior to the adjustment to the pressure.

**In** some examples, the processing system is configured to: continue to process the first and second signals to detect a change in sympathetic activity; and in response to the change in sympathetic activity failing to exceed the predetermined activity threshold, generate the second control signal configured to control the PAP device to provide air to the subject at a previous pressure.

**In** other words, once sympathetic activity has returned to normal levels, the PAP device is returned to its previous settings.

**In** some examples, the processing system is configured to repeatedly, at predetermined intervals: process the first and second signals to detect a change in sympathetic activity; in response to the detected change in sympathetic activity exceeding a predetermined activity threshold: process the first and second signals to determine whether the detected change in sympathetic activity is chemoreceptor-initiated or baroreceptor-initiated; determine an adjusted pressure to be provided by the PAP device, based on the determination as to whether the detected change in sympathetic activity is chemoreceptor-initiated or baroreceptor-initiated; and generate a first control signal configured to control the PAP device to provide air to the subject at the adjusted pressure.

This allows the pressure of the PAP device to be adjusted to provide an appropriate pressure throughout the sleep session, to keep sympathetic activity within normal levels. In this way, arousals may be reduced, and the sleep quality of the subject during the sleep session may be improved.

In some examples, the processing system is configured to: determine an initial adjusted pressure value based on the determination as to whether the detected change in sympathetic activity is chemoreceptor-initiated or baroreceptor-initiated; obtain a pressure threshold value; compare the initial adjusted pressure value and the pressure threshold value; in response to the initial adjusted pressure value exceeding the pressure threshold value, set the pressure threshold value as the adjusted pressure; and in response to the initial adjusted pressure value failing to exceed the pressure threshold value, set the initial adjusted pressure value as the adjusted pressure.

In other words, a limit on the maximum pressure provided to the subject by the PAP device may be established. The pressure threshold value may be a subject-specific value; for instance, a subject-specific pressure threshold value may be determined by a clinician during a titration session for the subject.

In some examples, the processing system is configured to obtain the pressure threshold value by: estimating a current sleep stage of the subject; and selecting the pressure threshold value, from a set of pressure threshold values, according to the estimated current sleep stage.

The inventors have recognized that subjects can tolerate higher levels of pressure (i.e. without arousal occurring) when in deep sleep than when in light sleep stages. By selecting a pressure threshold value according to an estimated current sleep stage, a higher pressure may, as required, be provided to the subject during deep sleep, while the pressure provided during light sleep is limited so as to reduce a likelihood of arousal.

The current sleep stage may be estimated by processing the first signal responsive to the subject's breathing, or, where used, a signal responsive to heart rate variability. Alternatively, the estimated sleep stage may be based on a current time of night or a number of hours for which the PAP device has been used in a current sleep session.

In some examples, wherein, in response to the initial adjusted pressure value exceeding the pressure threshold value, the processing system is further configured to generate a third control signal configured to control the PAP device to provide supplementary oxygen to the subject.

The provision of supplementary oxygen enables the reduction of SDB events at a lower pressure setting of the PAP device.

In some examples, the predetermined activity threshold is determined by: using a machine-learning algorithm trained to predict a likelihood of arousal for the subject based on changes in sympathetic activity; and wherein the predetermined activity threshold is set as a value of a change in sympathetic activity for which a likelihood of arousal predicted by the machine-learning algorithm exceeds a predetermined likelihood of arousal.

In other words, the predetermined threshold is a subject-specific threshold above which an arousal is likely if no action is taken.

In some examples, the processing system is configured to determine whether the detected change in sympathetic activity is chemoreceptor-initiated or baroreceptor-initiated by: processing the first and second signals to determine whether the detected change in sympathetic activity corresponds to a sleep disordered breathing event; in response to a determination that the detected change in sympathetic activity corresponds to a sleep disordered breathing event, determining that the detected change in sympathetic activity is chemoreceptor-initiated; in response to a determination that the detected change in sympathetic activity does not correspond to a sleep disordered breathing event, determining that the detected change in sympathetic activity is baroreceptor-initiated.

In other words, the presence of an SDB event immediately preceding a detected change in sympathetic activity indicates that the change is chemoreceptor-initiated, while the absence of an SDB event indicates that the change is baroreceptor-initiated.

In some examples, the processing system is configured to generate the control signal by: processing the determination as to whether the detected change in sympathetic activity is chemoreceptor-initiated or baroreceptor-initiated to determine a control instruction for controlling the SDB therapy device; and generating a fourth control signal configured to control an output user interface to provide a user-perceptible output of the control instruction.

In other words, rather than directly controlling the SDB therapy device, the processing system may provide a user with an instruction for controlling the SDB therapy device (e.g. an instruction to adjust one or more settings of the SDB therapy device).

There is also provided a system for controlling a sleep disordered breathing, SDB, therapy device providing therapy to a subject during a sleep session of the subject. The system comprises: one or more first sensors configured to generate the first signal responsive to the subject's breathing during the sleep session; one or more second sensors configured to generate the second signal responsive to baroreceptor-initiated changes in sympathetic activity of the subject; and the processing system described above, configured to receive the first and second signals from the one or more first sensors and one or more second sensors respectively.

The one or more first sensors may include an airflow sensor. The one or more second sensors may, for example, include a cardiac sensor, an electrodermal response sensor, a brain activity sensor, a muscle sensor, a pulse oximetry sensor, an exhaled breath sensor, a pressure sensor and/or an ultrasound sensor.

According to another aspect of the invention, there is provided a computer program product comprising computer program code means which, when executed on a computing device having a processing system, cause the processing system to perform all of the steps of a computer-implemented method for controlling a sleep disordered breathing, SDB, therapy device providing therapy to a subject during a sleep session of the subject, the computer-implemented method comprising: receiving a first signal responsive to the subject's breathing during the sleep session; receiving a second signal responsive to baroreceptor-initiated changes in sympathetic activity of the subject; processing the first and second signals to detect a change in sympathetic activity; in response to the detected change in sympathetic activity exceeding a predetermined activity threshold: processing the first and second signals to determine whether the detected change in sympathetic activity is chemoreceptor-initiated or baroreceptor-initiated; and generating a control signal for controlling the SDB therapy device, based on the determination as to whether the detected change in sympathetic activity is chemoreceptor-initiated or baroreceptor-initiated.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system for controlling a sleep disordered breathing (SDB) therapy device providing therapy to a subject during a sleep session of the subject, according to an embodiment of the invention;
Fig. 2 illustrates a system for controlling an SDB therapy device providing therapy to a subject during a sleep session of the subject, according to another embodiment of the invention; and
Fig. 3 illustrates a computer-implemented method for controlling an SDB therapy device providing therapy to a subject during a sleep session of the subject, the steps of the method being performed through a computer program product according to the present invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for controlling a sleep disordered breathing (SDB) therapy device. Signals responsive to a subject's breathing and baroreceptor-initiated changes in sympathetic activity of the subject, while the subject is undergoing therapy using the SDB therapy device, are obtained and processed to detect a change in sympathetic activity. For changes in sympathetic activity exceeding a predetermined threshold, a determination as to whether the change is chemoreceptor-initiated or baroreceptor-initiated. A control signal for controlling the SDB therapy device is generated according to whether the change is chemoreceptor-initiated or baroreceptor-initiated.

Embodiments are at least partly based on the realization that therapy intended to reduce sleep disordered breathing events may result in an increase in baroreceptor-initiated sympathetic activity, leading to an increase in arousals. By controlling the therapy according to whether a subject is experiencing chemoreceptor- or baroreceptor-initiated changes in sympathetic activity, a compromise between reducing sleep disordered breathing events and reducing baroreceptor-initiated arousals may be achieved, thus improving the subject's sleep quality and increasing a likelihood that the subject will continue with the SDB therapy.

Illustrative embodiments may, for example, be employed in sleep disordered breathing therapy systems, including positive airway pressure systems (e.g. APAP or BiPAP systems), mandibular advancement systems, and positional therapy systems.

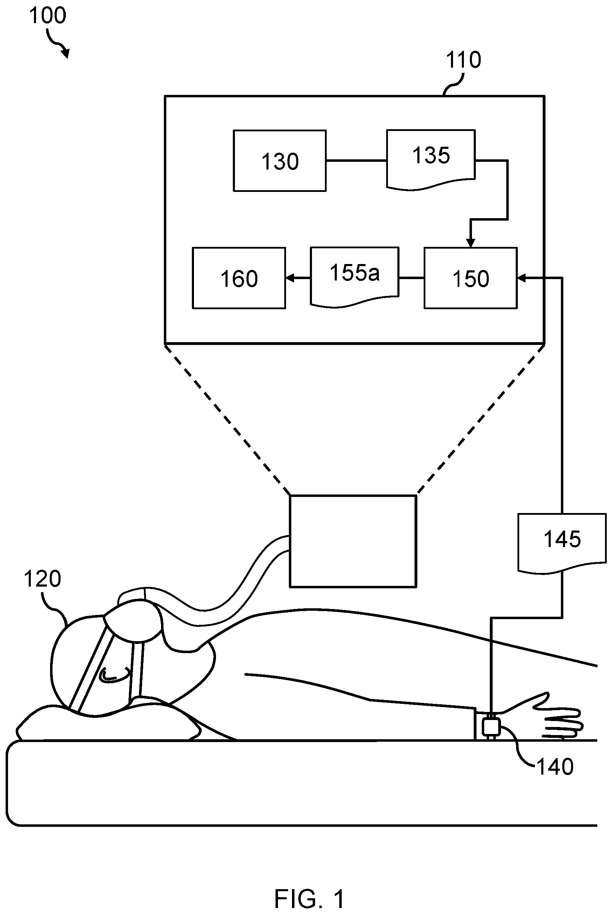

Fig. 1 illustrates a system 100 for controlling a sleep disordered breathing (SDB) therapy device 110 providing therapy to a subject 120 during a sleep session of the subject, according to an embodiment of the invention. The system 100 comprises one or more first sensors 130, one or more second sensors 140, and a processing system 150. The processing system 150 is, itself, an embodiment of the invention.

In Fig. 1, the SDB therapy device 110 is a positive airway pressure (PAP) device; however, as explained in more detail below, the system 100 may, in some embodiments, be used to control other types of SDB therapy devices, such as a mandibular advancement device (MAD) and/or a positional therapy device. The PAP device shown in Fig. 1 comprises a PAP machine that generates pressurized air, a mask that fits over the subject's nose and mouth, and a tube connecting the mask to the PAP machine; however, any PAP device may be used (e.g. having a CPAP machine, BiPAP machine or APAP machine, having a mask that fits only over the nose or mouth, etc.).

The subject 120 is preferably a subject having a sleep disordered breathing condition (e.g. OSA or CSA) and a cardiac comorbidity (e.g. hypertension, coronary artery disease, chronic heart failure, etc.).

In Fig. 1, the processing system 150 is provided within the PAP device 110; however, in some embodiments, the processing system 150 may be provided within a separate device to the SDB therapy device (e.g. the processing system may be configured to directly control the SDB therapy device remotely, and/or the processing system may control the SDB therapy device by providing a control instruction to an output user interface, as discussed below).

The one or more first sensors 130 are configured to generate a first signal 135 responsive to the subject's breathing during the sleep session. In Fig. 1, the one or more first sensors are provided within the PAP device, and comprise an airflow sensor. The inventors have advantageously recognized that airflow sensors are conventionally included in a PAP device, and therefore a signal responsive to a subject's breathing may be obtained for a subject using a PAP device without requiring additional sensors.

In some examples, the one or more first sensors 130 may additionally or alternatively include one or more sensors external to the SDB therapy device. Suitable sensors for generating a signal responsive to the subject's breathing (e.g. by measuring chest and/or abdominal movement and/or by measuring breathing sounds) will be apparent to the skilled person. For example, the one or more sensors may include at least one of: a microphone, an accelerometer, a gyroscope, an inertial measurement unit (IMU), a seismocardiography (SCG) sensor and/or a ballistocardiographic (BCG) sensor. The one or more first sensors may, as appropriate, be provided in the SDB therapy device (e.g. in the PAP machine of a PAP device and/or in the mask of a PAP device), in a wearable device worn by the subject 120 (e.g. a smartwatch, a chest belt and/or an abdominal belt) and/or in a device positioned close to the subject during the sleep session (e.g. a microphone of a subject's smartphone).

The one or more second sensors 140 are configured to generate a second signal 145 responsive to baroreceptor-initiated changes in sympathetic activity of the subject 120 during the sleep session. In Fig. 1, the one or more second sensors are provided within a smartwatch; however, the skilled person will readily appreciate that the one or more second sensors may additionally or alternatively be provided in other devices (e.g. other wearable devices, such as a chest belt, an abdominal belt, a finger-mounted device, a wearable patch, etc.). In some embodiments, one or more of the second sensor(s) may be provided in the SDB therapy device (e.g. in the mask of a PAP device).

The one or more second sensors 140 may be capable of measuring one or more of: cardiac activity (e.g. responsive to changes in heart rate variability), brain activity (e.g. responsive to activity associated with cortical arousals), muscle activity, a sympathetic skin response (e.g. responsive to changes in skin temperature, sweating and/or skin electrical impedance), a blood oxygen level, an exhaled CO₂ concentration and/or a change in intrathoracic pressure.

For example, cardiac activity (in particular, heart rate, heart rate variability and/or blood pressure) may be measured using an electrocardiography (ECG) sensor, a pulse oximetry sensor (e.g. a photoplethysmography (PPG) sensor), a ballistocardiography (BCG) sensor, a seismocardiography (SCG) sensor and/or a blood pressure sensor. Brain activity may be measured using an electroencephalography (EEG) sensor. Muscle activity may be measured using an electromyography (EMG) sensor. A sympathetic skin response may be measured using an electrodermal skin response sensor (also known as a galvanic skin response sensor). A blood oxygen level may be measured using a PPG sensor. An exhaled CO₂ concentration may be measured using an exhaled breath sensor.

Various types of sensors may be used to (indirectly) measure a change in intrathoracic pressure. For instance, the one or more second sensors 140 may obtain a surrogate measure of respiratory effort (e.g. a PPG sensor may be used to measure modulations of peripheral blood flow and blood accumulation) or a surrogate measure of neural respiratory drive (e.g. one or more surface EMG sensors may be used to measure electrical activity occurring in one or more muscles used for breathing). Other sensors that may be used to indirectly measure a change in intrathoracic pressure include a respiratory belt, a suprasternal notch pressure sensor and/or an ultrasound patch (e.g. to monitor diaphragm activity and/or cardiac output).

The processing system 150 is configured to receive the first signal 135 from the one or more first sensors 130 and the second signal 145 from the one or more second sensors 140, and process the first and second signals to detect a change in sympathetic activity. If no change in sympathetic activity is detected, the processing system may continue to receive and process the first and second signals until a change in sympathetic activity is detected.

A detected change in sympathetic activity may be a detected change in one or more parameters derivable from the first signal 135 and/or the second signal 145 that changes responsive to sympathetic activity. For instance, a detected change in sympathetic activity may, depending on the type(s) of sensors used to obtain the first and second signals, comprise one or more of: a change in respiratory rate, a change in tidal volume, a change in airflow pattern, a change in heart rate, a change in heart rate variability, a change in blood pressure, a change in sympathetic skin response, a change in brain activity indicative of cortical arousal, a change in muscle activity indicative of cortical arousal, a change in blood oxygen level, and/or a change in an exhaled CO₂ concentration.

Having detected a change in sympathetic activity, the processing system 150 determines whether the detected change in sympathetic activity exceeds a predetermined activity threshold. The predetermined activity threshold may be a threshold capable of distinguishing between changes in sympathetic activity that are too small to result in an arousal and changes in sympathetic activity that are likely to result in an arousal if no action is taken.

In some examples, the predetermined activity threshold may be a generic threshold that can be expected to distinguish between changes that are and are not likely to result in arousal for any subject. A generic predetermined activity threshold may be a relatively low threshold, so as to avoid excluding changes in sympathetic activity that may result in arousal in particularly light sleepers. A suitable value for the predetermined activity threshold will depend on the type of change in sympathetic activity detected (i.e. which parameter(s) derivable from the first and/or second signal a change has been detected in).

In some examples, the predetermined activity threshold (for a given parameter) may be determined according to the subject's phenotype. For instance, subjects may be grouped into phenotypes according to one or more of: age, body mass index, a type of comorbidity, a level of comorbidity, and/or a severity of sleep disordered breathing. Different predetermined activity thresholds may be determined for each phenotype.

In other examples, the predetermine activity threshold (for a given parameter) may be a subject-specific threshold determined during a titration session for the subject, by monitoring the physiological effects of changes in the SDB therapy provided to the subject during the titration session (e.g. changes in PAP settings).

In yet other examples, the predetermined activity threshold (for a given parameter) may be a subject-specific machine-learned threshold. For instance, the predetermined activity threshold may be determined by training a machine-learning algorithm to predict a likelihood of arousal for the subject based on changes in sympathetic activity. The predetermined activity threshold may then be set as a value of a change in sympathetic activity for which a likelihood of arousal predicted by the trained machine-learning algorithm exceeds a predetermined likelihood of arousal.

A machine-learning algorithm is any self-training algorithm that processes input data in order to produce or predict output data. Here, the input data comprises changes in sympathetic activity and the output data comprises a predicted likelihood of arousal if no action is taken.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to changes in sympathetic activity of the subject. The training output data entries correspond to information regarding whether or not each change in sympathetic activity resulted in arousal.

The training data may be obtained during one or more training sleep sessions. During the one or more training sleep sessions, an initial predetermined activity threshold may be applied. Alternatively, no action to control the SDB therapy device may be taken during the training sleep session(s) (i.e. the training sleep session may be used purely to collect data).

In some examples, the predetermined activity threshold may be iteratively modified during the one or more training sleep sessions until one or more predefined conditions are met. The one or more predetermined conditions may, for instance, relate to a number of hours of uninterrupted sleep or a number/frequency of arousals within a sleep session. For example, the predetermined activity threshold may be iteratively modified by the machine-learning algorithm, controlling the SDB therapy device responsive to changes in sympathetic activity exceeding a current value of the predetermined activity threshold as described below, until a period of four hours of uninterrupted sleep is achieved.

In response to the detected change in sympathetic activity exceeding the predetermined activity threshold, the processing system 150 is configured to process the first and second signals 135, 145 to determine whether the detected change in sympathetic activity is chemoreceptor-initiated or baroreceptor-initiated.

In some examples, the determination as to whether the detected change in sympathetic activity is chemoreceptor-initiated or baroreceptor-initiated is based on whether the detected change in sympathetic activity corresponds to a sleep disordered breathing (SDB) event (e.g an obstructive apnea or hypopnea event). For instance, a detected change in sympathetic activity may be determined to be chemoreceptor-initiated in response to a determination that the detected change corresponds to an SDB event. Similarly, a detected change in sympathetic activity may be determined to be baroreceptor-initiated in response to a determination that the detected change does not correspond to an SDB event.

In order to determine whether a detected change in sympathetic activity corresponds to an SDB event, the processing system 150 may be configured to process the first signal 135 to detect an SDB event. For example, the processing system may determine a value for one or more breathing parameters based on the first signal (e.g. a length of inspiration, a length of expiration, a rise time of a respiration cycle, a fall time of a respiration cycle, a tidal volume, a dynamic of inspiratory flow, a dynamic of expiratory flow, etc.), and process the value(s) to detect an SDB event.

Methods of detecting SDB events based on a signal responsive to a subject's breathing are known: see, for example, Nakano et al. (2007), "Automatic detection of sleep-disordered breathing from a single-channel airflow record", European Respiratory Journal, 29:728-736; Haidar et al. (2017), "Sleep apnea event detection from nasal airflow using convolutional neural networks", ICONIP 2017: Neural Information Processing, pp. 819-827; Yang et al. (2019), "Sleep apnea and hypopnea events detection based on airflow signals using LSTM network", 2019 41st Annual International Conference of the IEEE Engineering in Medicine and Biology Society (EMBC), pp. 2576-2579; and Koley and Dey (2013), "Real-time adaptive apnea and hypopnea event detection methodology for portable sleep apnea monitoring devices", IEEE Transactions on Biomedical Engineering, 60(12):3354-3363.

Additional physiological parameters (e.g. derivable from the second signal 145), such as cardiac parameters and/or a blood oxygen level, may be used in combination with the one or more breathing parameters to detect SDB events. Methods of detecting SDB events using additional physiological parameters in combination with one or more breathing parameters are known: see, for example, John et al. (2021), "SomnNET: An SpO2 based deep learning network for sleep apnea detection in smartwatches", 2021 43rd Annual International Conference of the IEEE Engineering in Medicine & Biology Society, pp. 1961-1964; and Saha et al. (2020), "Portable diagnosis of sleep apnea with the validation of individual event detection", Sleep Medicine, 69:51-57.

In response to a failure to detect an SDB event, the processing system 150 may determine that the detected change in sympathetic activity does not correspond to an SDB event and therefore determine that the change in sympathetic activity is baroreceptor-initiated.

In response to detecting an SDB event, the processing system 150 may determine a time between the detected SDB event and the detected change in sympathetic activity, and determine whether the change in sympathetic activity is chemoreceptor-initiated or baroreceptor-initiated based on whether the time between the SDB event and the change in sympathetic activity exceeds a predetermined time interval. A change in sympathetic activity may be determined to be baroreceptor-initiated in response to the time between the SDB event and the change in sympathetic activity exceeding the predetermined time interval, and chemoreceptor-initiated in response to the time between the SDB event and the change in sympathetic activity failing to exceed the predetermined time interval.

Baroreceptor-initiated changes in sympathetic activity tend to occur within a breath cycle of an SDB event, while chemoreceptor-initiated changes in sympathetic activity generally occur a few breath cycles after an SDB event (e.g. after about 5 breath cycles). Therefore, the predetermined time interval may be a time between 2 and 5 breath cycles (e.g. 3 breath cycles). The predetermined time interval may be determined based on a population average for a length of a breath cycle (e.g. based on an average breathing rate of 12-15 breaths per minute). Alternatively, an average breath cycle for the subject 120 may be determined based on the first signal 135, and used to determine the predetermined time interval.

In some examples, the processing system 150 may additionally or alternatively determine whether a detected change in sympathetic activity is chemoreceptor-initiated or baroreceptor-initiated based on whether the detected change comprises a change in oxygen saturation level and/or a change in a carbon dioxide concentration in the subject's exhaled air, where this information is available (i.e. where the one or more sensors comprise a PPG sensor and/or an exhaled breath sensor). For instance, in response to detecting a change in oxygen saturation in combination with an SDB event, the detected change in sympathetic activity may be determined as chemoreceptor-initiated. Similarly, a detected change in sympathetic activity may be determined as chemoreceptor-initiated in response to detecting a change in an exhaled CO₂ concentration in combination with an SDB event.

The processing system 150 is then configured to process the determination as to whether the detected change in sympathetic activity is chemoreceptor-initiated or baroreceptor-initiated to generate a control signal for controlling the SDB therapy device 110. The generated control signal may control the SDB therapy device directly and/or control an output user interface (not shown in Fig. 1) to output a control instruction for controlling the SDB therapy device.

In embodiments in which the SDB therapy device 110 is a PAP device, the control signal may be configured to control the pressure at which air is provided to the subject 120 by the PAP device based on whether the detected change in sympathetic activity is chemoreceptor-initiated or baroreceptor-initiated. In other words, the processing system 150 may be configured to process the determination as to whether the detected change in sympathetic activity is chemoreceptor-initiated or baroreceptor-initiated to determine an adjusted pressure for the PAP device, and generate a control signal 155a configured to control the PAP device to provide air to the subject at the adjusted pressure (e.g. by controlling the speed of a motor 160 of the PAP device).

For instance, in response to a determination that the detected change in sympathetic activity is chemoreceptor-initiated, the processing system 150 may determine that the pressure of air provided to the subject 120 should be increased, and determine a new, increased pressure at which to provide air to the subject. The processing system may then control the PAP device 110 to provide air to the subject at the new, increased pressure.

A chemoreceptor-initiated change in sympathetic activity is a change in sympathetic activity as a result of a respiratory deficiency. The decline in oxygen uptake and release of carbon dioxide caused by a respiratory deficiency triggers an activation of chemoreceptors. A chemoreceptor-initiated change in sympathetic activity thus indicates that a current pressure provided by the PAP is insufficient for treating the subject's sleep disordered breathing, and that a higher PAP pressure is required in order to reduce a likelihood of SDB events and chemoreceptor-initiated arousals, and thus to improve sleep quality.

Similarly, in response to a determination that the detected change in sympathetic activity is baroreceptor-initiated, the processing system 150 may determine that the pressure of air provided to the subject 120 should be decreased, and determine a new, decreased pressure at which to provide air to the subject. The processing system may then control the PAP device 110 to provide air to the subject at the new, decreased pressure.

A baroreceptor-initiated change in sympathetic activity is a change in sympathetic activity as a result of a change in intrathoracic pressure, which may be caused by a current pressure provided by the PAP device being excessively high. The change in cardiac parameters (including the venous return flow, atrial filling and stroke volume) caused by a change in intrathoracic pressure triggers an activation of baroreceptors. A baroreceptor-initiated change in sympathetic activity may thus indicate that a lower PAP pressure is required in order to reduce a likelihood of baroreceptor-initiated arousals and thus improve sleep quality.

In some examples, the pressure may be adjusted incrementally, until a desired effect is achieved. For instance, the processing system 150 may continue to receive and process the first and second signals 135, 145 while controlling the PAP device 110 to adjust the pressure in incremental steps, until the processing system determines that the sympathetic response has stabilized.

In some examples, having determined a suitable adjustment to the pressure for a particular change in sympathetic activity in this way, the processing system may record the determined adjusted pressure. The processing system may afterwards control the PAP device to provide air at this adjusted pressure in response to detecting the particular change in sympathetic activity (i.e. without requiring the incremental steps). In other words, the processing system may learn subject-specific pressure adjustments based on incrementally adjusting the pressure.

In some examples, an adjustment to the pressure to be made in response to a particular change in the sympathetic response of the subject may be determined during a titration session for the subject.

In some examples, a pressure threshold may be taken into account when determining the adjusted pressure (e.g. so that the adjusted pressure does not exceed a pressure threshold value). For instance, an initial adjusted pressure value may be determined based on the determination as to whether the detected change in sympathetic activity is chemoreceptor-initiated or baroreceptor-initiated, as described above. The initial adjusted pressure value may then be compared with a pressure threshold value: in response to the initial adjusted pressure value exceeding the pressure threshold value, the pressure threshold value is set as the adjusted pressure, while in response to the initial adjusted pressure value failing to exceed the pressure threshold value, the initial adjusted pressure is set as the adjusted pressure.

In other words, the pressure threshold value represents a maximum permitted pressure at which air may be provided to the subject 120 by the PAP device 110, for example, corresponding to a pressure at which a likely number of baroreceptor-initiated arousals exceeds a threshold. The pressure threshold value may, for example, be a subject-specific value determined by a clinician during a PAP titration session.

In some examples, the pressure threshold value may depend on a current sleep stage of the subject 120. For instance, the processing system 150 may estimate a current sleep stage of the subject, and select the pressure threshold value from a set of pressure threshold values according to the estimated current sleep stage. This recognizes that a subject can tolerate a higher pressure of PAP therapy (i.e. without experiencing baroreceptor-initiated arousals) during deeper sleep stages.

A current sleep stage of the subject may be estimated using time-based methods, e.g. based on a current time of night or a number of hours for which the PAP device 110 has been used in a current sleep session (either of which provides an estimate for a current number of hours of sleep).

Alternatively, a current sleep stage of the subject may be estimated by processing the first signal 135 and/or the second signal 145 using known methods for estimating sleep stage based on physiological signals. See, for example, Bakker et al. (2021), "Estimating sleep stages using cardiorespiratory signals: validation of a novel algorithm across a wide range of sleep-disordered breathing severity", J Clin Sleep Med., 17(7):1343-1354; Long et al. (2014), "Analyzing respiratory effort amplitude for automated sleep stage classification", Biomedical Signal Processing and Control, 14:197-205; Radha et al. (2019), "Sleep stage classification from heart-rate variability using long short-term memory neural networks", Scientific Reports, 9:14149; Beattie et al. (2017), "Estimation of sleep stages in a healthy adult population from optical plethysmography and accelerometer singals", Physiological Measurement, 38(11):1968-1979; Aboalayon et al. (2016), "Sleep stage classification using EEG signal analysis: A comprehensive survey and new investigation", Entropy, 18(9): 272; and IET Wireless Sensor Systems, 12(5-6):123-133; Imtiaz (2021), "A systematic review of sensing technologies for wearable sleep staging", Sensors, 21(5):1562.

Having estimated the current sleep stage, the processing system 150 may select the pressure threshold value corresponding to the estimated sleep stage from a set of pressure threshold values that comprises a pressure threshold values for each sleep stage. The pressure threshold values for each sleep stage may be subject-specific values determined by a clinician during a PAP titration session.

Where the initial adjusted pressure exceeds the pressure threshold value and the pressure threshold value is set as the adjusted pressure, the pressure provided to the subject 120 by the PAP device 110 may be insufficient to keep the number/frequency of SDB events below an acceptable (e.g. clinically-defined) level. In order to further reduce the number/frequency of SDB events without increasing the PAP pressure above the pressure threshold value, the processing system 150 may be configured to additionally control the PAP device 110 to provide supplementary oxygen to the subject in response to the initial adjusted pressure value exceeding the pressure threshold value. Supplementary oxygen improves oxygen saturation in subjects, thus enabling a reduction in SDB events without an increase in the pressure provided by the PAP device.

In some examples, the processing system 150 may be configured to control the PAP device 110 to return to a previous pressure in response to a predetermined condition bring met. In the context of the present disclosure, a "previous pressure" is a pressure at which air was provided to the subject 120 by the PAP device immediately prior to the adjustment of the pressure. For instance, the previous pressure may be a pressure prescribed to the subject by a clinician.

The predetermined condition may, for example, be time-dependent. For instance, the processing system 150 may control the PAP device 110 to provide air to the subject 120 at the adjusted pressure for a predetermined time period, and control the PAP device to provide air to the subject at the previous pressure in response to the predetermined time period elapsing. In some examples, the processing system 150 may control the PAP device to return to the previous pressure after a first adjustment to the pressure within a sleep session, then control the PAP device to maintain an adjusted pressure in response to a subsequent detection of a change in sympathetic activity and adjustment to the pressure.

In other examples, the predetermined condition may relate to the sympathetic activity of the subject 120. For instance, the processing system 150 may continue to process the first and second signals 135, 145 to detect a change in sympathetic activity while the PAP device 110 provides air to the subject at the adjusted pressure, and control the PAP device to provide air at the previous pressure in response to the detected change in sympathetic activity failing to exceed the predetermined activity threshold. Alternatively, the processing system may control the PAP device to provide air at the previous pressure in response to a determination that the physiological parameter for which the change in sympathetic activity was detected has returned to (within a predefined margin) a value of the physiological parameter before the change occurred. For instance, if the change in sympathetic activity is an increase in heart rate variability, the PAP device may be controlled to return to its previous pressure in response to the heart rate variability returning to or falling below the value of heart rate variability immediately preceding the increase.

In some examples, the processing system 150 may be configured to repeat, at predetermined intervals, the steps of processing the first and second signal 135, 145 to detect a change in sympathetic activity and, in response to the detected change in sympathetic activity exceeding a predetermined activity threshold, processing the first and second signals to determine whether the detected change in sympathetic activity is chemoreceptor-initiated or baroreceptor-initiated, determining an adjusted pressure based on whether the change is chemoreceptor-initiated or baroreceptor-initiated and generating a control signal to control the PAP device 110 to provide air to the subject 120 at the adjusted pressure.

The processing system 150 may repeat the above steps in addition to returning to a previous pressure (i.e. after the PAP device has returned to the previous pressure, in order to monitor for further changes in sympathetic pressure) or as an alternative (i.e. operating as a closed-loop feedback system).

By operating as a closed-loop feedback system, the processing system 150 may control the PAP device 110 in a way that takes into account the subject's response to the adjusted pressure. Due to the complexity of cardiopulmonary interactions, a change in sympathetic activity may not always be correctly classified as either chemoreceptor-initiated or baroreceptor-initiated, as respiratory deficiencies may induce a cardiac response and vice versa. In such cases, the pressure provided by the PAP device may be adjusted incorrectly (i.e. such that the adjusted pressure results in a negative subject response). By continuing to monitor changes in the subject's sympathetic activity and adjusting the pressure provided by the PAP device accordingly, a negative subject response may be corrected by re-adjusting the pressure.

The length of the predetermined interval may depend on whether the PAP device has returned to a previous pressure or whether the system is operating as a closed-loop feedback system, in which case the length of the predetermined interval may be determined according to an expected time taken for the subject to respond to a most recent adjustment made to the pressure provided by the PAP device. The length of the predetermined time interval may depend on a type of most recent pressure adjustment. For instance, a change made to the pressure in response to detecting a chemoreceptor-initiated change will take longer to have an observable effect on the subject (e.g. about 1 minute, i.e. the time taken to pump the subject's blood volume through the lungs) than a change made in response to detecting a baroreceptor-initiated change (which is typically observable in cardiac parameters within a few heartbeats).

Although the control signal 155a in Fig. 1 is shown in the context of controlling the pressure provided by a PAP device, the processing system 150 may additionally or alternatively be configured to directly control the therapy provided by other SDB therapy devices.

For instance, where the SDB therapy device is a positional therapy device, the processing system 150 may generate a control signal that controls the positional therapy device to change the subject's head and/or body position (e.g. by vibrating until a desired position is achieved). Where the SDB therapy device is a mandibular advancement device having motorized means for adjusting the jawbone protrusion, the processing system may generate a control signal that controls the motorized means to adjust the jawbone protrusion (e.g. increasing the jawbone protrusion in response to a chemoreceptor-initiated change in sympathetic activity, and decreasing the jawbone protrusion in response to a baroreceptor-initiated change).

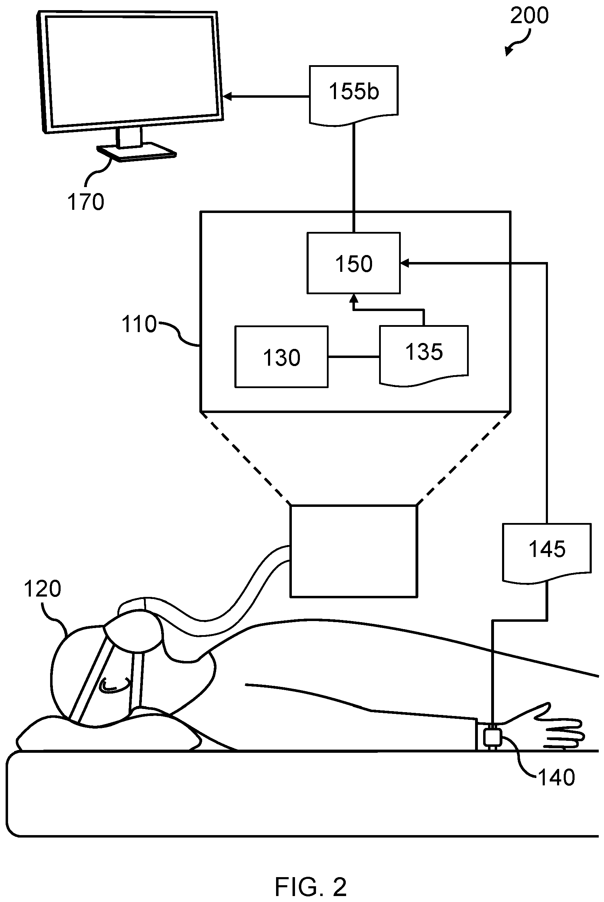

As previously mentioned, the control signal generated by the processing system 150 may control an output user interface. This is shown in Fig. 2, which illustrates a system 200 for controlling an SDB therapy device providing therapy to a subject during a sleep session of the subject, according to another embodiment of the invention.

System 200 is identical to the system 100 of Fig. 1, except that system 200 further comprises an output user interface 170, and the control signal is a control signal 155b for controlling the output user interface 170.

The processing system 150 may control the output user interface 170 by processing the determination as to whether the detected change in sympathetic activity is chemoreceptor-initiated or baroreceptor initiated to determine a control instruction for controlling the SDB therapy device, and generating a control signal 155b configured to control the output user interface to provide a user-perceptible output of the control instruction.

The control instruction determined by the processing system 150 will depend on the type of SDB therapy device to be controlled. For instance, in the case of a PAP device, the control instruction may be an instruction to adjust the pressure provided by the PAP device to a new, adjusted pressure determined by the processing system as described above. In the case of a mandibular advancement device, the control instruction may be an instruction to adjust the jawbone protrusion provided by the mandibular advancement device.

Suitable user-perceptible outputs for providing the control instruction to a user will be apparent to the skilled person, and may depend on the control instruction provided. For instance, the control instruction may be provided to the user in the form of a textual display and/or a sound (e.g. a spoken instruction). In some examples, the control instruction may be provided to the subject 120, while in other examples, the control instruction may be additionally or alternatively provided to a clinician. In Fig. 2, the output user interface 170 is a computer monitor; however, the skilled person will readily appreciate that any output user interface capable of providing a suitable user-perceptible output may be used (e.g. a mobile device or a speaker).

While the Figures illustrate the generation of control signal 155a and 155b as separate embodiments, in some examples, the processing system 150 may be configured to generate both a control signal for directly controlling the SDB therapy device 110 and a control signal for controlling an output user interface to provide a user-perceptible output of the control instruction.

In some examples, the processing system 150 may determine whether to generate a control signal for directly controlling the SDB therapy device 110 or a control signal for controlling the output user interface 170 based on one or more predefined rules. For instance, the processing system 150 may be configured to control a PAP device to provide air at an adjusted pressure provided that the adjusted pressure falls within a predefined pressure range, and configured to control an output user interface in response to the adjusted pressure not falling within the predefined pressure range. This reduces clinical intervention in modifying the PAP therapy provided to a subject, thus improving clinical efficiency, while allowing clinician input in cases where a recommended change in the adjusted pressure differs significantly from the pressure prescribed to the subject.

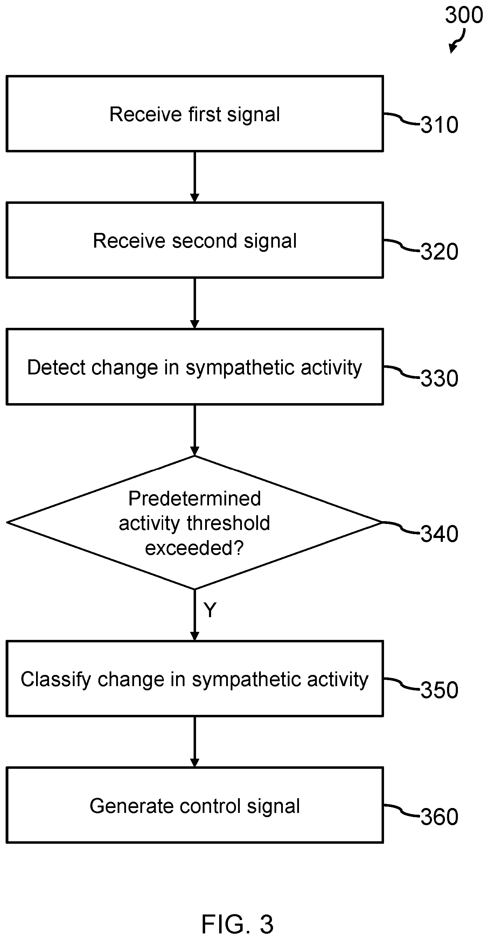

Fig. 3 illustrates a computer-implemented method 300 for controlling an SDB therapy device providing therapy to a subject during a sleep session of the subject, all the steps of the method being performed through a computer program product according to the present invention.

The method 300 begins at step 310, at which a first signal responsive to the subject's breathing during the sleep session is received.

At step 320, a second signal responsive to baroreceptor-initiated changes in sympathetic activity of the subject is received.

At step 330, the first and second signals are processed to detect a change in sympathetic activity.

At step 340, a determination is made as to whether the detected change in sympathetic activity exceeds a predetermined activity threshold.

In response to a determination that the detected change in sympathetic activity exceeds the predetermined activity threshold, the method proceeds to step 350, at which the first and second signals are processed to determine whether the detected change in sympathetic activity is chemoreceptor-initiated or baroreceptor-initiated.

At step 360, the determination as to whether the change in sympathetic activity is chemoreceptor-initiated or baroreceptor-initiated is processed to generate a control signal for controlling the SDB therapy device.

The disclosed methods are computer-implemented methods. As such, there is disclosed a computer program comprising code means for implementing any described method when said program is run on a processing system.

The skilled person would be readily capable of developing a processing system for carrying out any herein described method. Thus, each step of a flow chart may represent a different action performed by a processing system, and may be performed by a respective module of the processing system.

As discussed above, the system makes use of a processing system to perform the data processing. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs). Thus, the processing system may be embodied as a digital and/or analog processing system.

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A processing system (150) for controlling a sleep disordered breathing, SDB, therapy device (110) providing therapy to a subject (120) during a sleep session of the subject, the processing system being configured to:
receive a first signal (135) responsive to the subject's breathing during the sleep session;
receive a second signal (145) responsive to baroreceptor-initiated changes in sympathetic activity of the subject;
process the first and second signals to detect a change in sympathetic activity;
in response to the detected change in sympathetic activity exceeding a predetermined activity threshold:
process the first and second signals to determine whether the detected change in sympathetic activity is chemoreceptor-initiated or baroreceptor-initiated; and
generate a control signal (155a, 155b) for controlling the SDB therapy device, based on the determination as to whether the detected change in sympathetic activity is chemoreceptor-initiated or baroreceptor-initiated.

2. The processing system (150) of claim 1, wherein:
the SDB therapy device (110) is a positive airway pressure, PAP, device;
wherein the control signal comprises a first control signal (155a), and
wherein the processing system is configured to:
determine an adjusted pressure to be provided by the PAP device, based on the determination as to whether the detected change in sympathetic activity is chemoreceptor-initiated or baroreceptor-initiated; and
generate the first control signal (155a) to control the PAP device to provide air to the subject (120) at an adjusted pressure.

3. The processing system (150) of claim 2, wherein:
in response to a determination that the detected change in sympathetic activity is chemoreceptor-initiated, the adjusted pressure is an increased pressure; and
in response to a determination that the detected change in sympathetic activity is baroreceptor-initiated, the adjusted pressure is a decreased pressure.

4. The processing system (150) of claim 2 or 3, wherein the processing system is configured to:
generate the first control signal (155a) configured to control the PAP device (110) to provide air to the subject (120) at the adjusted pressure for a predetermined time period; and
in response to the predetermined time period elapsing, generate a second control signal configured to control the PAP device to provide air to the subject at a previous pressure.

5. The processing system (150) of claim 2 or 3, wherein the processing system is configured to:
continue to process the first and second signals (135, 145) to detect a change in sympathetic activity; and
in response to the change in sympathetic activity failing to exceed the predetermined activity threshold, generate the second control signal configured to control the PAP device (110) to provide air to the subject (120) at a previous pressure.

6. The processing system (150) of any of claims 2 to 5, wherein the processing system is configured to repeatedly, at predetermined intervals:
process the first and second signals (135, 145) to detect a change in sympathetic activity;
in response to the detected change in sympathetic activity exceeding a predetermined activity threshold:
process the first and second signals to determine whether the detected change in sympathetic activity is chemoreceptor-initiated or baroreceptor-initiated;
determine an adjusted pressure to be provided by the PAP device, based on the determination as to whether the detected change in sympathetic activity is chemoreceptor-initiated or baroreceptor-initiated; and
generate a first control signal (155a) configured to control the PAP device (110) to provide air to the subject (120) at the adjusted pressure.

7. The processing system (150) of any of claims 2 to 6, configured to:
determine an initial adjusted pressure value based on the determination as to whether the detected change in sympathetic activity is chemoreceptor-initiated or baroreceptor-initiated;
obtain a pressure threshold value;
compare the initial adjusted pressure value and the pressure threshold value;
in response to the initial adjusted pressure value exceeding the pressure threshold value, set the pressure threshold value as the adjusted pressure; and
in response to the initial adjusted pressure value failing to exceed the pressure threshold value, set the initial adjusted pressure value as the adjusted pressure.

8. The processing system (150) of claim 7, configured to obtain the pressure threshold value by:
estimating a current sleep stage of the subject (120); and
selecting the pressure threshold value, from a set of pressure threshold values, according to the estimated current sleep stage.

9. The processing system (150) of claim 7 or 8, wherein, in response to the initial adjusted pressure value exceeding the pressure threshold value, the processing system is further configured to generate a third control signal configured to control the PAP device (110) to provide supplementary oxygen to the subject (120).

10. The processing system (150) of any of claims 1 to 9, wherein the predetermined activity threshold is determined by:
using a machine-learning algorithm trained to predict a likelihood of arousal for the subject (120) based on changes in sympathetic activity; and
wherein the predetermined activity threshold is set as a value of a change in sympathetic activity for which a likelihood of arousal predicted by the machine-learning algorithm exceeds a predetermined likelihood of arousal.

11. The processing system (150) of any of claims 1 to 10, configured to determine whether the detected change in sympathetic activity is chemoreceptor-initiated or baroreceptor-initiated by:
processing the first and second signals (135, 145) to determine whether the detected change in sympathetic activity corresponds to a sleep disordered breathing event;
in response to a determination that the detected change in sympathetic activity corresponds to a sleep disordered breathing event, determining that the detected change in sympathetic activity is chemoreceptor-initiated;
in response to a determination that the detected change in sympathetic activity does not correspond to a sleep disordered breathing event, determining that the detected change in sympathetic activity is baroreceptor-initiated.

12. The processing system (150) of any of claims 1 to 11, configured to generate the control signal (155a, 155b) by:
processing the determination as to whether the detected change in sympathetic activity is chemoreceptor-initiated or baroreceptor-initiated to determine a control instruction for controlling the SDB therapy device (110); and
generating a fourth control signal (155b) configured to control an output user interface (170) to provide a user-perceptible output of the control instruction.

13. A system (100) for controlling a sleep disordered breathing, SDB, therapy device (110) providing therapy to a subject (120) during a sleep session of the subject, the system comprising:
one or more first sensors (130) configured to generate the first signal (135) responsive to the subject's breathing during the sleep session;
one or more second sensors (140) configured to generate the second signal (145) responsive to baroreceptor-initiated changes in sympathetic activity of the subject; and
the processing system (155) of any of claims 1 to 12, configured to receive the first and second signals from the one or more first sensors and one or more second sensors respectively.

14. A computer program product comprising computer program code means which, when executed on a computing device having a processing system, cause the processing system to perform all of the steps of a computer-implemented method (300) for controlling a sleep disordered breathing, SDB, therapy device (110) providing therapy to a subject (120) during a sleep session of the subject, the computer-implemented method comprising:
receiving a first signal (135) responsive to the subject's breathing during the sleep session;
receiving a second signal (145) responsive to baroreceptor-initiated changes in sympathetic activity of the subject;
processing the first and second signals to detect a change in sympathetic activity;
in response to the detected change in sympathetic activity exceeding a predetermined activity threshold:
processing the first and second signals to determine whether the detected change in sympathetic activity is chemoreceptor-initiated or baroreceptor-initiated; and
generating a control signal (155a, 155b) for controlling the SDB therapy device, based on the determination as to whether the detected change in sympathetic activity is chemoreceptor-initiated or baroreceptor-initiated.

15. The computer program product of claim 14, wherein:
the SDB therapy device (110) is a positive airway pressure, PAP, device;
wherein the control signal comprises a first control signal (155a), and
wherein the computer-implemented method comprises:
determining an adjusted pressure to be provided by the PAP device, based on the determination as to whether the detected change in sympathetic activity is chemoreceptor-initiated or baroreceptor-initiated; and
generating the first control signal (155a) to control the PAP device to provide air to the subject (120) at an adjusted pressure.

## Patentansprüche

1. Verarbeitungssystem (150) zum Steuern einer Therapievorrichtung (110) für schlafbezogene Atmungsstörungen, SDB, die einem Subjekt (120) während einer Schlafsitzung des Subjekts Therapie bereitstellt, wobei das Verarbeitungssystem dazu konfiguriert ist:
ein erstes Signal (135) in Reaktion auf die Atmung des Subjekts während der Schlafsitzung zu empfangen;
ein zweites Signal (145) in Reaktion auf barorezeptor-initiierte Änderungen der Sympathikusaktivität des Subjekts zu empfangen;
das erste und das zweite Signal zu verarbeiten, um eine Änderung der Sympathikusaktivität zu erkennen;
in Reaktion darauf, dass die erkannte Änderung der Sympathikusaktivität eine vorbestimmte Aktivitätsschwelle überschreitet:
das erste und das zweite Signal zu verarbeiten, um zu bestimmen, ob die erkannte Änderung der Sympathikusaktivität chemorezeptor-initiiert oder barorezeptor-initiiert ist; und
ein Steuersignal (155a, 155b) zum Steuern der SDB-Therapievorrichtung auf Basis der Bestimmung, ob die erkannte Änderung der Sympathikusaktivität chemorezeptor-initiiert oder barorezeptor-initiiert ist, zu erzeugen.

2. Verarbeitungssystem (150) nach Anspruch 1, wobei:
die SDB-Therapievorrichtung (110) eine Vorrichtung mit positivem Atemwegsdruck, PAP, ist;
wobei das Steuersignal ein erstes Steuersignal (155a) umfasst, und
wobei das Verarbeitungssystem dazu konfiguriert ist:
einen angepassten Druck, der von der PAP-Vorrichtung bereitgestellt werden soll, auf Basis der Bestimmung, ob die erkannte Änderung der Sympathikusaktivität chemorezeptor-initiiert oder barorezeptor-initiiert ist, zu bestimmen; und
das erste Steuersignal (155a) zu erzeugen, um die PAP-Vorrichtung so zu steuern, dass sie dem Subjekt (120) Luft mit einem angepassten Druck bereitstellt.

3. Verarbeitungssystem (150) nach Anspruch 2, wobei:
in Reaktion auf eine Bestimmung, dass die erkannte Änderung der Sympathikusaktivität chemorezeptor-initiiert ist, der angepasste Druck ein erhöhter Druck ist; und
in Reaktion auf eine Bestimmung, dass die erkannte Änderung der Sympathikusaktivität barorezeptor-initiiert ist, der angepasste Druck ein verminderter Druck ist.

4. Verarbeitungssystem (150) nach Anspruch 2 oder 3, wobei das Verarbeitungssystem dazu konfiguriert ist:
das erste Steuersignal (155a) zu erzeugen, das dazu konfiguriert ist, die PAP-Vorrichtung (110) so zu steuern, dass sie dem Subjekt (120) Luft mit dem angepassten Druck über eine vorbestimmte Zeitspanne bereitstellt; und
in Reaktion darauf, dass die vorbestimmte Zeitspanne abläuft, ein zweites Steuersignal zu erzeugen, das dazu konfiguriert ist, die PAP-Vorrichtung so zu steuern, dass sie dem Subjekt Luft mit einem vorherigen Druck bereitstellt.

5. Verarbeitungssystem (150) nach Anspruch 2 oder 3, wobei das Verarbeitungssystem dazu konfiguriert ist:
das erste und das zweite Signal (135, 145) weiter zu verarbeiten, um eine Änderung der Sympathikusaktivität zu erkennen; und
in Reaktion darauf, dass die Änderung der Sympathikusaktivität die vorbestimmte Aktivitätsschwelle nicht überschreitet, das zweite Steuersignal zu erzeugen, das dazu konfiguriert ist, die PAP-Vorrichtung (110) so zu steuern, dass sie dem Subjekt (120) Luft mit einem vorherigen Druck bereitstellt.

6. Verarbeitungssystem (150) nach einem der Ansprüche 2 bis 5, wobei das Verarbeitungssystem dazu konfiguriert ist, wiederholt, in vorbestimmten Intervallen:
das erste und das zweite Signal (135, 145) zu verarbeiten, um eine Änderung der Sympathikusaktivität zu erkennen;
in Reaktion darauf, dass die erkannte Änderung der Sympathikusaktivität eine vorbestimmte Aktivitätsschwelle überschreitet:
das erste und das zweite Signal zu verarbeiten, um zu bestimmen, ob die erkannte Änderung der Sympathikusaktivität chemorezeptor-initiiert oder barorezeptor-initiiert ist;
einen angepassten Druck, der von der PAP-Vorrichtung bereitgestellt werden soll, auf Basis der Bestimmung, ob die erkannte Änderung der Sympathikusaktivität chemorezeptor-initiiert oder barorezeptor-initiiert ist, zu bestimmen; und
ein erstes Steuersignal (155a) zu erzeugen, das dazu konfiguriert ist, die PAP-Vorrichtung (110) so zu steuern, dass sie dem Subjekt (120) Luft mit dem angepassten Druck bereitstellt.

7. Verarbeitungssystem (150) nach einem der Ansprüche 2 bis 6, das dazu konfiguriert ist:
einen anfänglichen angepassten Druckwert auf Basis der Bestimmung, ob die erkannte Änderung der Sympathikusaktivität chemorezeptor-initiiert oder barorezeptor-initiiert ist, zu bestimmen;
einen Druckschwellenwert zu erhalten;
den anfänglichen angepassten Druckwert und den Druckschwellenwert zu vergleichen;
in Reaktion darauf, dass der anfängliche angepasste Druckwert den Druckschwellenwert überschreitet, den Druckschwellenwert als angepassten Druck einzustellen; und
in Reaktion darauf, dass der anfängliche angepasste Druckwert den Druckschwellenwert nicht überschreitet, den anfänglichen angepassten Druckwert als angepassten Druck einzustellen.

8. Verarbeitungssystem (150) nach Anspruch 7, das dazu konfiguriert ist, den Druckschwellenwert zu erhalten durch:
Schätzen einer aktuellen Schlafphase des Subjekts (120); und
Auswählen des Druckschwellenwerts aus einem Satz Druckschwellenwerte gemäß der geschätzten aktuellen Schlafphase.

9. Verarbeitungssystem (150) nach Anspruch 7 oder 8, wobei das Verarbeitungssystem weiter dazu konfiguriert ist, in Reaktion darauf, dass der anfängliche angepasste Druckwert den Druckschwellenwert überschreitet, ein drittes Steuersignal zu erzeugen, das dazu konfiguriert ist, die PAP-Vorrichtung (110) so zu steuern, dass sie dem Subjekt (120) zusätzlichen Sauerstoff bereitstellt.

10. Verarbeitungssystem (150) nach einem der Ansprüche 1 bis 9, wobei die vorbestimmte Aktivitätsschwelle bestimmt wird durch:
Verwenden eines maschinellen Lernalgorithmus, der darauf trainiert ist, eine Erregungswahrscheinlichkeit für das Subjekt (120) auf Basis von Änderungen der Sympathikusaktivität vorherzusagen; und
wobei die vorbestimmte Aktivitätsschwelle als Wert einer Änderung der Sympathikusaktivität eingestellt wird, bei dem eine von dem maschinellen Lernalgorithmus vorhergesagte Erregungswahrscheinlichkeit eine vorbestimmte Erregungswahrscheinlichkeit überschreitet.

11. Verarbeitungssystem (150) nach einem der Ansprüche 1 bis 10, das dazu konfiguriert ist, zu bestimmen, ob die erkannte Änderung der Sympathikusaktivität chemorezeptor-initiiert oder barorezeptor-initiiert ist durch:
Verarbeiten des ersten und des zweiten Signals (135, 145), um zu bestimmen, ob die erkannte Änderung der Sympathikusaktivität einem Ereignis einer schlafbezogenen Atmungsstörung entspricht;
in Reaktion auf eine Bestimmung, dass die erkannte Änderung der Sympathikusaktivität einem Ereignis einer schlafbezogenen Atmungsstörung entspricht, zu bestimmen, dass die erkannte Änderung der Sympathikusaktivität chemorezeptor-initiiert ist;
in Reaktion auf eine Bestimmung, dass die erkannte Änderung der Sympathikusaktivität keinem Ereignis einer schlafbezogenen Atmungsstörung entspricht, zu bestimmen, dass die erkannte Änderung der Sympathikusaktivität barorezeptor-initiiert ist.

12. Verarbeitungssystem (150) nach einem der Ansprüche 1 bis 11, das dazu konfiguriert ist, das Steuersignal (155a, 155b) zu erzeugen durch:
Verarbeiten der Bestimmung, ob die erkannte Änderung der Sympathikusaktivität chemorezeptor-initiiert oder barorezeptor-initiiert ist, um eine Steueranweisung zum Steuern der SDB-Therapievorrichtung (110) zu bestimmen; und
Erzeugen eines vierten Steuersignals (155b), das dazu konfiguriert ist, eine Ausgabebenutzerschnittstelle (170) so zu steuern, dass sie eine vom Benutzer wahrnehmbare Ausgabe der Steueranweisung bereitstellt.

13. System (100) zum Steuern einer Therapievorrichtung (110) für schlafbezogene Atmungsstörungen, SDB, die einem Subjekt (120) während einer Schlafsitzung des Subjekts Therapie bereitstellt, wobei das System umfasst:
einen oder mehrere erste Sensoren (130), die dazu konfiguriert sind, das erste Signal (135) in Reaktion auf die Atmung des Subjekts während der Schlafsitzung zu erzeugen;
einen oder mehrere zweite Sensoren (140), die dazu konfiguriert sind, das zweite Signal (145) in Reaktion auf barorezeptor-initiierte Änderungen der Sympathikusaktivität des Subjekts zu erzeugen; und
das Verarbeitungssystem (155) nach einem der Ansprüche 1 bis 12, das dazu konfiguriert ist, das erste und das zweite Signal von dem einen oder den mehreren ersten Sensoren bzw. dem einen oder den mehreren zweiten Sensoren zu empfangen.

14. Computerprogrammprodukt, das Computerprogrammcodemittel umfasst, die, wenn sie auf einer Rechenvorrichtung ausgeführt werden, die ein Verarbeitungssystem aufweist, das Verarbeitungssystem dazu bringen, alle Schritte eines computerimplementierten Verfahrens (300) zum Steuern einer Therapievorrichtung (110) für schlafbezogene Atmungsstörungen, SDB, die einem Subjekt (120) während einer Schlafsitzung des Subjekts eine Therapie bereitstellt, durchzuführen, wobei das computerimplementierte Verfahren umfasst:
Empfangen eines ersten Signals (135) in Reaktion auf die Atmung des Subjekts während der Schlafsitzung;
Empfangen eines zweiten Signals (145) in Reaktion auf barorezeptor-initiierte Änderungen der Sympathikusaktivität des Subjekts;
Verarbeiten des ersten und des zweiten Signals, um eine Änderung der Sympathikusaktivität zu erkennen;
in Reaktion darauf, dass die erkannte Änderung der Sympathikusaktivität eine vorbestimmte Aktivitätsschwelle überschreitet:
Verarbeiten des ersten und des zweiten Signals, um zu bestimmen, ob die erkannte Änderung der Sympathikusaktivität chemorezeptor-initiiert oder barorezeptor-initiiert ist; und
Erzeugen eines Steuersignals (155a, 155b) zum Steuern der SDB-Therapievorrichtung auf Basis der Bestimmung, ob die erkannte Änderung der Sympathikusaktivität chemorezeptor-initiiert oder barorezeptor-initiiert ist.

15. Computerprogrammprodukt nach Anspruch 14, wobei:
die SDB-Therapievorrichtung (110) eine Vorrichtung mit positivem Atemwegsdruck, PAP, ist;
wobei das Steuersignal ein erstes Steuersignal (155a) umfasst, und wobei das computerimplementierte Verfahren umfasst:
Bestimmen eines angepassten Drucks, der von der PAP-Vorrichtung bereitgestellt werden soll, auf Basis der Bestimmung, ob die erkannte Änderung der Sympathikusaktivität chemorezeptor-initiiert oder barorezeptor-initiiert ist; und
Erzeugen des ersten Steuersignals (155a), um die PAP-Vorrichtung so zu steuern, dass sie dem Subjekt (120) Luft mit einem angepassten Druck bereitstellt.

## Revendications

1. Système de traitement (150) permettant de commander un dispositif de thérapie des troubles respiratoires du sommeil (TRS) (110) fournissant une thérapie à un sujet (120) pendant une séance de sommeil du sujet, le système de traitement étant configuré pour :
recevoir un premier signal (135) sensible à la respiration du sujet pendant la séance de sommeil ;
recevoir un deuxième signal (145) sensible à des changements initiés par barorécepteur d'activité sympathique du sujet ;
traiter les premier et deuxième signaux pour détecter un changement d'activité sympathique ;
en réponse au changement détecté d'activité sympathique dépassant un seuil d'activité prédéterminé :
traiter les premier et deuxième signaux pour déterminer si le changement détecté d'activité sympathique est initié par chimiorécepteur ou par barorécepteur ; et
générer un signal de commande (155a, 155b) pour commander le dispositif de thérapie TRS, en fonction de la détermination selon laquelle le changement détecté d'activité sympathique est initié par chimiorécepteur ou par barorécepteur.

2. Système de traitement (150) selon la revendication 1, dans lequel :
le dispositif de thérapie TRS (110) est un dispositif à pression positive des voies aériennes, PAP ;
dans lequel le signal de commande comprend un premier signal de commande (155a), et
dans lequel le système de traitement est configuré pour :
déterminer une pression ajustée à fournir par le dispositif PAP, en fonction de la détermination selon laquelle le changement détecté d'activité sympathique est initié par chimiorécepteur ou par barorécepteur ; et
générer le premier signal de commande (155a) pour commander le dispositif PAP afin de fournir de l'air au sujet (120) à une pression ajustée.

3. Système de traitement (150) selon la revendication 2, dans lequel :
en réponse à une détermination selon laquelle le changement détecté d'activité sympathique est initié par chimiorécepteur, la pression ajustée est une pression augmentée ; et
en réponse à une détermination selon laquelle le changement détecté d'activité sympathique est initié par barorécepteur, la pression ajustée est une pression diminuée.

4. Système de traitement (150) selon la revendication 2 ou 3, dans lequel le système de traitement est configuré pour :
générer le premier signal de commande (155a) configuré pour commander le dispositif PAP (110) afin de fournir de l'air au sujet (120) à la pression ajustée pendant une période de temps prédéterminée ; et
en réponse à l'expiration de la période de temps prédéterminée, générer un deuxième signal de commande configuré pour commander le dispositif PAP afin de fournir de l'air au sujet à une pression précédente.

5. Système de traitement (150) selon la revendication 2 ou 3, dans lequel le système de traitement est configuré pour :
continuer à traiter les premier et deuxième signaux (135, 145) pour détecter un changement d'activité sympathique ; et
en réponse au changement d'activité sympathique ne dépassant pas le seuil d'activité prédéterminé, générer le deuxième signal de commande configuré pour commander le dispositif PAP (110) afin de fournir de l'air au sujet (120) à une pression précédente.

6. Système de traitement (150) selon l'une quelconque des revendications 2 à 5, dans lequel le système de traitement est configuré pour, de manière répétée, à des intervalles prédéterminés :
traiter les premier et deuxième signaux (135, 145) pour détecter un changement d'activité sympathique ;
en réponse au changement détecté d'activité sympathique dépassant un seuil d'activité prédéterminé :
traiter les premier et deuxième signaux pour déterminer si le changement détecté d'activité sympathique est initié par chimiorécepteur ou par barorécepteur ;
déterminer une pression ajustée à fournir par le dispositif PAP, en fonction de la détermination selon laquelle le changement détecté d'activité sympathique est initié par chimiorécepteur ou par barorécepteur ; et
générer un premier signal de commande (155a) configuré pour commander le dispositif PAP (110) afin de fournir de l'air au sujet (120) à la pression ajustée.

7. Système de traitement (150) selon l'une quelconque des revendications 2 à 6, configuré pour :
déterminer une valeur de pression ajustée initiale en fonction de la détermination selon laquelle le changement détecté d'activité sympathique est initié par chimiorécepteur ou par barorécepteur ;
obtenir une valeur seuil de pression ;
comparer la valeur de pression ajustée initiale à la valeur seuil de pression ;
en réponse à la valeur de pression ajustée initiale dépassant la valeur seuil de pression, définir la valeur seuil de pression comme pression ajustée ; et
en réponse à la valeur de pression ajustée initiale ne dépassant pas la valeur seuil de pression, définir la valeur de pression ajustée initiale comme pression ajustée.

8. Système de traitement (150) selon la revendication 7, configuré pour obtenir la valeur seuil de pression par :
l'estimation du stade de sommeil actuel du sujet (120) ; et
la sélection de la valeur seuil de pression, parmi un ensemble de valeurs seuil de pression, en fonction du stade de sommeil actuel estimé.

9. Système de traitement (150) selon la revendication 7 ou 8, dans lequel, en réponse à la valeur de pression ajustée initiale dépassant la valeur de seuil de pression, le système de traitement est en outre configuré pour générer un troisième signal de commande configuré pour commander le dispositif PAP (110) afin de fournir de l'oxygène supplémentaire au sujet (120).

10. Système de traitement (150) selon l'une quelconque des revendications 1 à 9, dans lequel le seuil d'activité prédéterminé est déterminé par :
l'utilisation d'un algorithme d'apprentissage automatique entraîné pour prédire une probabilité d'éveil du sujet (120) sur la base de changements d'activité sympathique ; et
dans lequel le seuil d'activité prédéterminé est défini comme une valeur d'un changement d'activité sympathique pour lequel une probabilité d'éveil prédite par l'algorithme d'apprentissage automatique dépasse une probabilité d'éveil prédéterminée.

11. Système de traitement (150) selon l'une quelconque des revendications 1 à 10, configuré pour déterminer si le changement détecté d'activité sympathique est initié par chimiorécepteur ou par barorécepteur par :
le traitement des premier et deuxième signaux (135, 145) pour déterminer si le changement détecté d'activité sympathique correspond à un événement de troubles respiratoires du sommeil ;
en réponse à une détermination selon laquelle le changement détecté d'activité sympathique correspond à un événement de troubles respiratoires du sommeil, la détermination que le changement détecté d'activité sympathique est initié par chimiorécepteur ;
en réponse à une détermination selon laquelle le changement détecté d'activité sympathique ne correspond pas à un événement de troubles respiratoires du sommeil, la détermination que le changement détecté d'activité sympathique est initié par barorécepteur.

12. Système de traitement (150) selon l'une quelconque des revendications 1 à 11, configuré pour générer le signal de commande (155a, 155b) par :
le traitement de la détermination selon laquelle le changement détecté d'activité sympathique est initié par chimiorécepteur ou par barorécepteur pour déterminer une instruction de commande pour commander le dispositif de thérapie TRS (110) ; et
la génération d'un quatrième signal de commande (155b) configuré pour commander une interface utilisateur de sortie (170) afin de fournir une sortie perceptible par l'utilisateur de l'instruction de commande.

13. Système (100) permettant de commander un dispositif de thérapie des troubles respiratoires du sommeil (TRS) (110) fournissant une thérapie à un sujet (120) pendant une séance de sommeil du sujet, le système comprenant :
un ou plusieurs premiers capteurs (130) configurés pour générer le premier signal (135) en réponse à la respiration du sujet pendant la séance de sommeil ;
un ou plusieurs seconds capteurs (140) configurés pour générer le deuxième signal (145) en réponse à des changements initiés par barorécepteurs d'activité sympathique du sujet ; et
le système de traitement (155) selon l'une quelconque des revendications 1 à 12, configuré pour recevoir les premier et deuxième signaux provenant respectivement du ou des premiers capteurs et du ou des seconds capteurs.

14. Produit de programme informatique comprenant un code de programme informatique qui, lorsqu'il est exécuté sur un dispositif informatique présentant un système de traitement, amène le système de traitement à réaliser toutes les étapes d'un procédé mis en œuvre par ordinateur (300) pour commander un dispositif de thérapie des troubles respiratoires du sommeil (TRS) (110) fournissant une thérapie à un sujet (120) pendant une séance de sommeil du sujet, le procédé mis en œuvre par ordinateur comprenant :
la réception d'un premier signal (135) sensible à la respiration du sujet pendant la séance de sommeil ;
la réception d'un deuxième signal (145) sensible à des changements initiés par barorécepteur d'activité sympathique du sujet ;
le traitement des premier et deuxième signaux pour détecter un changement d'activité sympathique ;
en réponse au changement détecté d'activité sympathique dépassant un seuil d'activité prédéterminé :
le traitement des premier et deuxième signaux pour déterminer si le changement détecté d'activité sympathique est initié par chimiorécepteur ou par barorécepteur ; et
la génération d'un signal de commande (155a, 155b) pour commander le dispositif de thérapie TRS, sur la base de la détermination selon laquelle le changement détecté d'activité sympathique est initié par chimiorécepteur ou par barorécepteur.

15. Produit de programme informatique selon la revendication 14, dans lequel :
le dispositif de thérapie TRS (110) est un dispositif à pression positive des voies aériennes, PAP ;
dans lequel le signal de commande comprend un premier signal de commande (155a), et dans lequel le procédé mis en œuvre par ordinateur comprend :
la détermination d'une pression ajustée à fournir par le dispositif PAP, sur la base de la détermination selon laquelle le changement détecté d'activité sympathique est initié par chimiorécepteur ou par barorécepteur ; et
la génération du premier signal de commande (155a) pour commander le dispositif PAP afin de fournir de l'air au sujet (120) à une pression ajustée.
